# EUROPÄISCHE PATENTSCHRIFT

(11) **EP 1 912 621 B1**
(45) Veröffentlichungstag und Bekanntmachung des Hinweises auf die Patenterteilung: **11.09.2019**
(21) Anmeldenummer: 06761235.8
(22) Anmeldetag: 21.07.2006
(51) Int. Cl.: A61K 9/00, A61K 9/14, A61K 31/33, A61K 31/195, A61K 38/04, A61K 47/00, A61K 47/42

(54) **PHARMAZEUTISCHE ZUSAMMENSETZUNG ENTHALTEND INDOMETACIN UND/ODER ACEMETACIN**
PHARMACEUTICAL COMPOSITION CONTAINING INDOMETACIN AND/OR ACEMETACIN
COMPOSITION PHARMACEUTIQUE A TENEUR EN INDOMETACINE ET/OU EN ACEMETACINE

(30) Priorität: 02.08.2005 CH 12812005; 01.12.2005 CH 19102005
(43) Veröffentlichungstag der Anmeldung: 23.04.2008
(73) Patentinhaber: Drossapharm AG, 4002 Basel (CH)
(72) Erfinder: IMBODEN, Roger, CH-4142 Münchenstein (CH); ROTHENBÜHLER, Erich, CH-4104 Oberwill (CH); LUTZ, Juerg, CH-4102 Binningen (CH)
(74) Vertreter: Braun, André jr.
(86) Internationale Anmeldenummer: PCT/CH2006/000384
(87) Internationale Veröffentlichungsnummer: WO 2007/014476

(56) Entgegenhaltungen:
- WO-A-98/47534
- US-A- 4 687 762
- US-A- 5 932 245
- US-A1- 2003 119 909
- DATABASE WPI Section Ch, Week 198445 Derwent Publications Ltd., London, GB; Class B02, AN 1984-279241 XP002365109 & JP 59 172415 A (POLA KASEI KOGYO KK) 29. September 1984 (1984-09-29)

## Beschreibung

Die vorliegende Anmeldung betrifft pharmazeutische Zusammensetzungen bzw. Darreichungsformen, welche mindestens einen der Wirkstoffe Indometacin oder Acemetacin in mikronisierter Form enthalten, wie in Anspruch 1 beschrieben.

Die Verbindungen Indometacin und Acemetacin sowie deren Herstellung sind bekannt. Die Verbindungen haben entzündungshemmende, schmerzlindernde und fiebersenkende Eigenschaften. Indometacin wird chemisch als 1-(p-Chlorbenzoyl)-5-methoxy-2-methyl-3-indolyl-essigsäure bezeichnet. Acemetacin wird chemisch als 1-(p-Chlorbenzoyl)-5-methoxy-2-methyl-3-indolyl-acetoxyessigsäure bezeichnet.

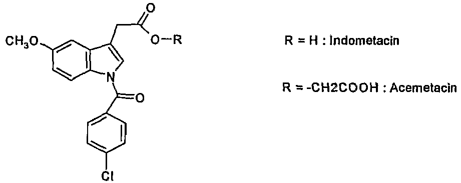

Die genannten Wirkstoffe, insbesondere Acemetacin, sind hydrophob, schlecht benetzbar und auch schlecht löslich in für die pharmazeutische Verwendung geeigneten Lösungsmitteln. Zudem haben die beiden Wirkstoffe, insbesondere Acemetacin, einen bitteren Geschmack, was diese für die perorale Anwendung, beispielsweise in Form einer Brausezubereitung, ungeeignet macht. Wider Erwarten liess sich der bittere Geschmack, beispielsweise in einer Brausezubereitung oder einer Suspension mit den üblicherweise in der Pharmazie verwendeten Geschmack- und Aromastoffen nicht überdecken. Bei der Verwendung der genannten Wirkstoffe, beispielsweise bei chronischen und insbesondere bei akuten Schmerzzuständen, ist aber eine möglichst schnelle Freisetzung des Wirkstoffs, bzw. die schnelle Erreichung eines hohen Blutspiegelwertes, und fallweise auch die perorale Verwendung, beispielsweise in Form einer Brausezubereitung, von grossem Vorteil.

US 4,687,762 beschreibt die Herstellung von wasserlöslichen Komplexen von in Wasser unlöslichen pharmazeutisch aktiven Verbindungen, wie z.B. Acemetacin, indem man die pharmazeutischaktive Verbindung in einem organischen Lösungsmittel löst und mit einem Phospholipid in Kontakt bringt. Dieses Verfahren hat den Nachteil, dass ein organisches Lösungsmittel verwendet werden muss, wobei auch die ursprüngliche kristalline Form aufgelöst wird und eine liposomale Formulierung als halbfeste Vesikelform erhalten wird. US 5,932,245 beschreibt eine kolloidale Dispersion, welche mit ausgewählter Gelatine hergestellt wird, u.a. für Acemetacin und Indometacin, in Form eines Nanosols. Es wurde nun gefunden, dass die Wirkstoffe Indometacin und Acemetacin, insbesondere Acemetacin, in mikronisierter Form, also wie in Anspruch 1 beschrieben, die genannten Nachteile nicht oder nur in reduzierter Form aufweisen. Derart können diese Wirkstoffe überraschenderweise in schnell löslichen, bzw. schnell freisetzenden galenischen Darreichungsformen peroral verabreicht werden, insbesondere in Form von Brausezubereitungen, Suspensionen oder in schnell löslichen festen Arzneiformen, wie Tabletten oder Kapseln, was in kurzer Zeit für eine medizinische Anwendung ausreichend hohe Blutspiegelwerte ergibt, was besonders in der Behandlung von chronischen und insbesondere akuten Schmerzzuständen von Vorteil ist. Hinzu kommt der weitere Vorteil, dass durch die Abspaltung von Kohlendioxid bei Brausezubereitungen im Magen ein Reiz auf die Magenwände entsteht, welcher peristaltische Bewegungen auslöst, was zu einem erwünschten raschen Abtransport der Brausezubereitungs-Lösung, resp. der Brausezubereitungs-Suspension, in das Doduenum führt.

Weiter wurde gefunden, dass der bittere Geschmack von mikronisiertem Indometacin und Acemetacin, überraschenderweise durch den Zusatz von Flavonoid-Derivaten, wie in Anspruch 1 unter (iv) definiert, bereits in geringen Mengen effizient maskiert wird. Überraschend ist auch, dass trotz der grossen Oberfläche des mikronisiertem Wirkstoffs und der damit verbundenen erhöhten geschmacklichen Aktivität, mit vergleichsweise kleinen Konzentrationen von Flavonoid-Derivaten eine effiziente Geschmacksmaskierung erreicht wird.

Die vorliegende Erfindung betrifft eine pharmazeutisch wirksame Zusammensetzung bzw. pharmazeutische Darreichungsform, gemäss Anspruch 1, welche mindestens einen der Wirkstoffe Indometacin oder Acemetacin sowie gegebenenfalls weitere Zusatzstoffe enthält, dadurch gekennzeichnet, dass diese Zusammensetzung den Wirkstoff oder ein Gemisch dieser Wirkstoffe, d.h. Indometacin und/oder Acemetacin, in mikronisierter Form enthält.

Die vorliegende Erfindung betrifft im Weiteren eine vorgehend definierte Zusammensetzung bzw. pharmazeutische Darreichungsform, welche dadurch gekennzeichnet ist, dass diese den Wirkstoff oder die Wirkstoffe im Gemisch mit mindestens einem Flavonoid-Derivat enthält.

Die vorliegende Erfindung betrifft auch Verfahren zur Herstellung der erfindungsgemässen Zusammensetzung, welches dadurch gekennzeichnet ist, dass man den Wirkstoff oder die Wirkstoffe vorgehend zur Herstellung der Darreichungsform, vorzugsweise unter Verwendung mechanischer Mittel, mikronisiert, d.h. mechanisch mikronisiert, und anschliessend unter Verwendung des mikronisierten Wirkstoffs oder der mikronisierten Wirkstoffe die Darreichungsform herstellt.

Die vorliegende Erfindung betrifft auch die Verwendung der erfindungsgemässen Zusammensetzung zur Behandlung von Schmerzzuständen, Entzündungen und Fieber, insbesondere Chronische Polyarthritis, Degenerative Gelenkerkrankungen, insbesondere der großen Gelenke und der Wirbelsäule, Bechterew-Erkrankung, Gicht, Entzündliche Zustände der Gelenke, Muskeln und Sehnen, Sehnenscheidenentzündungen, Schleimbeutelentzündungen, Hexenschuss (Lumbago), Entzündungen oberflächlicher Venen (Thrombophlebitis).

Die vorliegende Erfindung betrifft auch die Wirkstoffe Indometacin oder Acemetacin als loses Pulver, gegebenenfalls im Gemisch mit weiteren Zusatzstoffen, dadurch gekennzeichnet, dass die Wirkstoffe in mikronisierter Form vorliegen.

Die vorliegende Erfindung betrifft auch die Wirkstoffe Indometacin oder Acemetacin als loses Pulver, gegebenenfalls im Gemisch mit weiteren Zusatzstoffen, dadurch gekennzeichnet, dass die Wirkstoffe in mikronisierter Form im Gemisch mit mindestens einem Flavonoid-Derivat vorliegen.

Die vorliegende Erfindung betrifft auch die Verwendung der Wirkstoffe Indometacin oder Acemetacin in mikronisierter Form als loses Pulver im Gemisch mit einem Flavonoid-Derivat sowie gegebenenfalls im Gemisch mit weiteren Zusatzstoffen, für die Herstellung der erfindungsgemässen normal und schnell freisetzenden Darreichungsformen bzw. von pharmazeutischen Zusammensetzungen, insbesondere für die medizinische Behandlung von Schmerzzuständen, Entzündungen und Fieber, insbesondere Chronische Polyarthritis, Degenerative Gelenkerkrankungen, insbesondere der großen Gelenke und der Wirbelsäule, Bechterew-Erkrankung, Gicht, Entzündliche Zustände der Gelenke, Muskeln und Sehnen, Sehnenscheidenentzündungen, Schleimbeutelentzündungen, Hexenschuss (Lumbago), Entzündungen oberflächlicher Venen (Thrombophlebitis).

Die beanspruchten pharmazeutische Zusammensetzungen bzw. pharmazeutische Darreichungsformen umfassen insbesondere (i) Tabletten, wie Peroraltabletten, Kautabletten, Oraltabletten (Lutsch-, Sublingual-, Bukkaltabletten), Lösungstabletten, Brausetabletten; (ii) Kapseln, wie Hartgelatinekapseln und Weichgelatinekapseln; (iii) flüssige Arzneiformen, wie Lösungen, Emulsionen sowie Suspensionen. Im Sinne der vorliegenden Erfindung bedeutet "mikronisiert" eine feinste Partikelgrösse im Mikrometerbereich, wobei diese mikronisierten Partikel erfindungsgemäss vorzugsweise mittels mechanischer Mittel hergestellt werden und deren Kristallstruktur unverändert erhalten bleibt. Vorzugsweise bedeutet der Ausdruck "Wirkstoff in mikronisierter Form", dass mindestens 90 Vol.-%, vorzugsweise mindestens 95 Vol.-%, und vorzugsweise mindestens 98 Vol.-% des Wirkstoffs, eine Korngrösse von weniger als 25 µm (Mikron) (<25 µm) und vorzugsweise weniger als 21 µm (Mikron) (<21 µm), aufweist. In diesem Sinne liegt der "Wirkstoff in mikronisierter Form" vorzugsweise in Form von Mikrokristallen vor, mit welchem auch hochdisperse Systeme hergestellt werden können.

Vorzugsweise haben mindestens 50 Vol.-% und vorzugsweise mindestens 60 Vol.-% des Wirkstoffs, eine Korngrösse von weniger als 10 µm (Mikron) (<10 µm), vorzugsweise weniger als 8µm (Mikron) (<8 µm).

Vorzugsweise haben mindestens 30 Vol.-% des Wirkstoffs und vorzugsweise mindestens 50 Vol.-% des Wirkstoffs eine Korngrösse von weniger als 5 µm (Mikron) (<5 µm). Die untere Grenze der Korngrösse liegt jeweils für alle genannten Werte bei etwa 1 µm (Mikron). Dabei wird der mikronisierte Wirkstoff entweder als mikronisiertes Pulver oder in verarbeiteter Form, beispielsweise als Pellet oder Granulat, direkt verwendet oder weiter verarbeitet. Methoden für die Mikronisierung von Wirkstoffen sind an sich bekannt. Solche Methoden sind beispielsweise die Trockenmahlung in einer Kugelmühle oder Luftstrahlmühle und die Nassmahlung in einer Rührwerkskugelmühle (Perlmühle, Sandmühle) oder Kolloidmühle, wie solche in der einschlägigen Fachliteratur beschrieben sind. Die für die Mikronisierung verwendbaren Apparaturen sind kommerziell erhältlich, zum Beispiel die Apparatur Chrispro® Jet-Mill MC 300KX-TD der Firma Micro-Macinazione SA. Bevorzugt ist die Luftstrahlmahlung (jet milling).

Beispiele für Flavonoid-Derivate sind Chalcon- und Dihydrochalconglycoside. Solche Verbindungen sind an sich bekannt.. Typische Vertreter sind z.B. Naringinchalcon (R = Glycosid, 3,4-ungesättigt) bzw. Hesperitin-dihydrochalconglucosid (R = Glucosid), Neohesperidin-dihydrochalcon (R = Glycosid), insbesondere Neohesperidin-dihydrochalcon. Dihydrochalcone entsprechend der folgenden Formel: 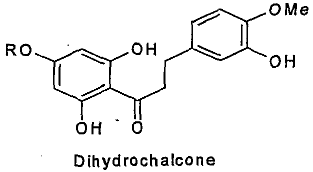 worin R ein an sich bekannter Rest bedeutet, vorzugsweise Wasserstoff oder (C₁₋₆)-Alkyl. Vorzugsweise bedeutet R einem Rest entsprechend der in Weiteren aufgeführten Dihydrochalconverbindungen.

Das Gewichtsverhältnis von Wirkstoff zur Flavonoidverbindung liegt vorzugsweise im Bereich von 10 : 1 bis 50 : 1, vorzugsweise im Bereich von 2 : 1 bis 50 : 1, insbesondere im Bereich 1 : 1 bis 15 : 1. Im Falle von Neohesperidin-dihydrochalcon insbesondere bei etwa 6 : 1. Dabei wird der Wirkstoff oder werden die Wirkstoffe mit der Flavonoidverbindung oder einem Gemisch solcher Verbindungen gemischt, wobei der Wirkstoff gegebenenfalls in an sich bekannter Weise mikroverkapselt wird.

Beispiele für gegebenenfalls im Gemisch zusätzlich zu den Chalcon- und Dhydrochalconglycosiden anwesende Oligopeptide und Polypeptide sowie davon abgeleiteten Derivate sind insbesondere Dipeptide, wie die von L-Asparaginsäure abgeleiteten Dipeptide und von L-Asparaginsäure abgeleiteten Dipeptidester; von L-Aminomalonsäure abgeleitete Dipeptide und Dipeptidester; insbesondere L-Aspartyl-D-Alanin, L-Aspartyl-L-phenylalanin-methylester, L-Aspartyl-L-Methionin-methylester, sowie von Lysin abgeleitete Dipeptide und Dipeptidester, insbesondere N-Phenylacetylglycyllysin, N-Acetyl-phenylalanyl-lysin, sowie aus tropischen Pflanzen gewonnene Polypeptide und Polypeptidgemische mit einer Molmasse zwischen 5 und 100 kDa mit hoher Süsskraft, wie Brazzein (Monomer: etwa 54 Aminosäuren), Curculin (Monomer: etwa 114 Aminosäuren), Mabinlin (Dimer: etwa 33 + etwa 72 Aminosäuren), Miraculin (Tetramer: etwa 191 Aminosäuren), Monellin (Dimer: etwa 45 + etwa 50 Aminosäuren), Pentadin und Thaumatin (etwa 207 Aminosäuren). Enthält die Zusammensetzung ein Gemisch von Flavonoid und Polypeptid so beträgt das Mischungsverhältnis Flavonoid : Polypeptid vorzugsweise 3 : 1 bis 1 : 3, vorzugsweise 2 : 1 bis 1 : 2 und vorzugsweise im Bereich von 1 : 1. So sind Mischungsverhältnisse von Flavonoid : Polypeptid von 1 : 3 oder 1 : 2 oder 1 : 1 oder 2 : 1 oder 3 : 1 problemlos, wobei die Gesamtmenge von Flavonoid + Polypeptid der oben für eine einzelne der beiden Komponenten angegebenen Menge entspricht.

Für die Herstellung von Tabletten, wie Peroraltabletten, Kautabletten, Oraltabletten (Lutsch-, Sublingual-, Bukkaltabletten), Lösungstabletten, Brausetabletten, verwendet man an sich bekannte Techniken, wie die Direkttablettierung oder die Tablettierung nach vorgängiger Herstellung von Granulaten oder Pellets. Dabei können übliche in der Zusammensetzung inerte Zusatzstoffe verwendet werden.

Als Zusatzstoffe für diese Granulate, Pellets und Tabletten sind beispielsweise verwendbar: Füllmittel, wie z.B. an sich bekannte Stärkearten, Lactose, Cellulosen, Mannitol, Sorbitol; Bindemittel, wie z.B. an sich bekannte Stärken, Cellulosen, Polyethylenglykole; Sprengmittel, wie z.B. an sich für diesen Zweck verwendbare an sich bekannte Stärken, Cellulosen, Alginate, Polyvinylpyrrolidone, Natriumhydrogencarbonat; Schmiermittel, wie z.B. Stearate, wie Magnesiumstearat; Fliessreguliermittel, wie z.B. Siliziumdioxid; sowie an sich bekannte Filmbildner.

Als Stärken sind an sich alle Stärken verwendbar, wobei Reis-, Weizen-, und Kartoffelstärken bevorzugt sind. Ebenso sind an sich bekannte modifizierte Stärken, einsetzbar, wie mit Methyl, Hydroxymethyl und/oder Hydroxypropyl modifizierte Stärken. Diese werden vorzugsweise in Anteilen von etwa 5-20% bezogen auf das Gesamtgewicht der Darreichungsform eingesetzt.

Als Cellulosen sind unmodifizierte pulverförmige Cellulose, mikrokristalline Cellulose, oder modifizierte Cellulose, wie mit Methyl, Ethyl, Propyl, Hydroxymethyl, Hydroxyethyl und/oder Hydroxypropyl modifizierte Cellulosen, wie z.B. HPMC, verwendbar. Diese können vorzugsweise im Bereich von 20 bis 90 Gew.-%, bezogen auf das Gesamtgewicht der Darreichungsform, eingesetzt oder auch als Beschichtungsmaterial verwendet werden.

Siliziumdioxid wird vorzugsweise in kolloidaler Form, in Mengen von etwa 0.1 bis 0.5%, bezogen auf das Gewicht aller Zusatzstoffe, eingesetzt. Lactose kann in unbehandelter Form, oder z.B. sprühgetrocknet, in Mengen im Bereich von etwa 65-85%, bezogen auf das Gesamtgewicht der Zusatzstoffe, eingesetzt werden. Polyethylenglykole und deren Derivate werden als Schmiermittel oder Bindemittel in Mengen von 0.5 - 5 Gew.-%, bezogen auf das Gesamtgewicht der Zusatzstoffe, eingesetzt. Polyvinylpyrrolidone, in Mengen von 0.5 - 5 Gew.-%, bezogen auf das Gesamtgewicht der Zusatzstoffe, können als Bindemittel, als Sprengmittel oder als Beschichtungsmittel verwendet werden. Calciumhydrogenphosphate und Mannitol werden als Verdünnungsmittel im Bereich von 10 - 90 Gew.-%, bezogen auf das Gesamtgewicht der Zusatzstoffe, eingesetzt. Stearate, vorzugsweise Magnesiumstearat, wird als Schmiermittel im Bereich von 0.25 - 5 Gew.-%, bezogen auf das Gesamtgewicht aller Zusatzstoffe, eingesetzt.

Die Herstellung von Brausezubereitungen, z.B. Brausetabletten, ist an sich bekannt. Diese können die vorgehend für die Herstellung von Tabletten genannten Zusatzstoffe enthalten, weisen jedoch zusätzlich einen Brausekörper auf. Dieser Brausekörper besteht in der Regel aus einer Kombination eines Karbonats oder Hydrogenkarbonats, vorzugsweise Natriumhydrogencarbonat, einerseits, mit einer geeigneten organischen Säure, vorzugsweise Zitronensäure oder Ascorbinsäure, andererseits. Solche Zusätze als Brausekörper sind an sich bekannt und in zahlreichen Zusammensetzungen beschrieben.

Für die vorliegende Erfindung ist die Zusammensetzung des Brausekörper unkritisch. Bevorzugt ist ein Brausekörper bestehend aus Natriumhydrogenkarbonat und Zitronensäure und/oder Ascorbinsäure.

In diesem Sinne betrifft die vorliegende Erfindung auch eine Brausezubereitung, vorzugsweise eine Brausetablette, welche mindestens einen der Wirkstoffe Indometacin oder Acemetacin, vorzugsweise Acemetacin, sowie weitere für eine Brausezubereitung an sich bekannte Zusatzstoffe enthält, dadurch gekennzeichnet, dass diese Zusammensetzung den Wirkstoff oder die Wirkstoffe, d.h. Indometacin und/- oder Acemetacin, in mikronisierter Form und vorzugsweise im Gemisch mit mindestens einem Flavonoid-Derivat und ggf. einem Polypeptid enthält.

Dabei gelten die vorgehend angegebene Definitionen, für die Korngrössenverteilung des mikronisierten Wirkstoffs oder der mikronisierten Wirkstoffe und deren Herstellung, für das Flavonoid-Derivat oder das Polypeptid, sowie die weiteren Zusatzstoffe, wobei diese Wirkstoffe in der Brausezubereitung, vorzugsweise in der Brausetablette, in pulveriger Form oder in freisetzungsmodifizierter Form, z.B. als Pellets, oder als Pellets, welche mit einem Filmüberzug versehen sind, vorliegen können.

Bevorzugt ist eine Brausezubereitung, vorzugsweise eine Brausetablette, welche aus (a) einem Wirkstoffgranulat enthaltend den mikronisierten Wirkstoff, welcher (b) im Gemisch mit mindestens einem Flavonoid-Derivat und ggf. einem Polypeptid vorliegt, (c) einen Brausekörper bestehend aus mindestens einer silikonisierten anorganischen Carbonatverbindung oder Hydrocarbonatverbindung, wie silikonisiertes Natriumhydrogencarbonat, und/oder silikonisiertes Calciumcarbonat und aus einer organischen Säure, vorzugsweise Zitronensäure, sowie gegebenenfalls (d) weiteren Zusatzstoffen, besteht.

Zusatzstoffe sind beispielsweise ausgewählt aus der Gruppe enthaltend Süssstoffe, synthetische Zuckerersatzstoffe sowie deren Salze, Polyole, natürliche und synthetisch hergestellte Aromastoffe, Beschwerungsmittel, Tenside, Farbstoffe, Füllmittel, und Bindemittel.

Beispiele für Zusatzstoffe sind Süssstoffe, wie Polysaccharide, wie z.B. Saccharose, Sucrose, Fructose, Glucose, Dextrose, Isomalt, Inulin, Lactitol, Trehalose, synthetische Zuckerersatzstoffe, sowie deren Salze, wie Xylit, Cyclamat, Acesulfam, Sucralose, Saccharin, Alitame, Aspartam; Polyole, wie z.B. Glycerol, Mannitol, Vanillin; Zitronenaroma, Erdbeeraroma, an sich bekannte natürliche und synthetisch hergestellte Aromastoffe. Das Wirkstoffgranulat kann ein Beschwerungsmittel, z.B. Natriumsulfat, und/oder Bariumsulfat; ein Tensid, z.B Polyoxyethylen Rizinusöl-Derivat, wie Macrogol-Glycerylhydroxystearat, Polyoxyethylenstearat, wie Polyoxyl-8-stearat, Polyoxyethylen Sorbitan Fettsäureester, wie Polyoxyethylene-20-sorbitan-monostearate oder Poloxamere und einen Farbstoff, enthalten.

Der Brausekörper kann zusätzlich Füllmittel in einer Menge von vorzugsweise 15 Gew.-% bis 30 Gew.-%, insbesondere 26.2% (bezogen auf die Gesamtmasse der Brausezubereitung) enthalten. Solche Füllmittel sind beispielsweise Lactose und/oder Sorbit, in unbehandelter oder vorbehandelter, z.B. granulierter Form. Der Brausekörper kann zusätzlich ein Bindemittel in einer Menge vorzugsweise im Bereich von 0.2 Gew.-% bis 0.6 Gew.-%, insbesondere 0.43% (bezogen auf das Gesamtgewicht der Brausezubereitung), vorzugsweise Polyvinylpyrrolidon (PVP), vorzugsweise in einer Menge von 0.6 Gew.-% bis 1 Gew.-%, bezogen auf Brausekörper, und einem Farbstoff enthalten.

Die Carbonatverbindung, vorzugsweise siliconisiertes Natriumhydrogencarbonat, wird vorzugsweise separat hergestellt, unter Zusatz von Silicon-Antischaumemulsion zum Natriumhydrogencarbonat (typischerweise etwa 0.32 Gew.-% berechnet auf das Gewicht des Natriumhydrogencarbonats), wobei das molare Verhältnis von Natriumhydrogencarbonat zur Säure vorzugsweise etwa 1 : 1 beträgt.

Eine effiziente, lang anhaltende Maskierung des bitteren Wirkstoffgeschmacks wird durch den Einsatz der vorgenannten Geschmacksmaskierer erreicht, wie dies vorgehend beschrieben ist.

Eine optimale Geschmacksmaskierung wird durch eine Kombination von zwei oder mehreren Süssstoffen erreicht. So können beispielsweise eine Kombination von zwei Süssstoffen, wie ein Flavonoid-Derivat, z.B. Neohesperidin, mit einem Polypeptid, z.B. L-Aspartyl-D-Alanin, eingesetzt werden. Eine, gegebenenfalls zusätzliche, spontane, kurz anhaltende Süssigkeit kann z.B. mit Natriumsaccharinat, Mannitol, Maltitol und/oder Sorbitol, vorzugsweise mit Natriumsaccharinat, erreicht werden. Eine langsam eintretende, länger anhaltende Süssigkeit kann mit Aspartam, Xylitol und/oder Alitam, vorzugsweise mit Aspartam, erreicht werden.

Insbesondere perorale Darreichungsformen, welche im Mund geschmacklich wirksam sind, wie Brausezubereitungen, Suspensionen, im Munde zerfallende Tabletten, sowie auch allgemein andere Darreichungsformen, können Geschmackskorrigentien enthalten, wie beispielsweise ätherische Öle; organische Säuren, wie z.B. Zitronensäure oder Ascorbinsäure; Polysaccharide, wie z.B. Saccharose, Sucrose, Fructose, Glucose, Dextrose, Isomalt, Inulin, Lactitol, Trehalose; synthetische Zuckerersatzstoffe, sowie deren Salze, wie Xylitol, Cyclamat, Acesulfam, Sucralose, Saccharin, Alitame, Aspartam; Polyole, wie z.B. Glycerol, Sorbitol, Mannitol; Vanillin.

Die Herstellung von Kapseln, wie Hartgelatinekapseln und Weichgelatinekapseln; von flüssigen Arzneiformen, wie Lösungen, Emulsionen sowie Suspensionen; ist an sich bekannt und kann mit an sich bekannten Zusatzstoffen vorgenommen werden. Die Herstellung von Hartgelatinekapseln und Weichgelatinekapseln ist an sich bekannt, wobei die erfindungsgemässe Zusammensetzung in an sich bekannter Weise in Hartgelatinekapseln, vorzugsweise als Granulat, abgefüllt oder in Weichgelatinekapseln, vorzugsweise als viskose Suspension oder als Paste, verarbeitet wird. Solche Granulate, viskose Suspensionen und Pasten können vom Fachmann nach analogen Rezepturen ohne weiteres hergestellt werden. Eine bevorzugte Ausführungsform für erfindungsgemäss verwendbare Pellets besteht darin, dass diese Pellets (a) mindestens einen der Wirkstoffe Indometacin oder Acemetacin oder ein Gemisch derselben in, vorzugsweise durch mechanische Mittel erreichter, mikronisierter Form und zusätzlich ein Bindemittel sowie ein Beschwerungsmittel enthalten, und gegebenenfalls (b) diese Pellets mit einem magensaft-resistenten Lack überzogen sind und/oder in Gegenwart eines magensaftresisten Lacks hergestellt worden sind. Vorzugsweise weisen diese Pellets eine scheinbare Dichte von 1,4-2,4 g/cm³, vorzugsweise von 1,5-1,8 g/cm³, vorzugsweise 1,5-1,8 g/cm³, auf. Deren Durchmesser liegt vorzugsweise im Bereich von 0,2-1,8 mm, wobei die Pellet-Zubereitung vorzugsweise etwa 0,1-80 Gew.-% Wirkstoff, vorzugsweise etwa 20-95 Gew.-%, vorzugsweise etwa 40-80 Gew.%, Beschwerungsmittel und im Weiteren Bindemittel, Farbstoff und Säuerungsmittel ad 100 Gew.-% enthält. Als Beschwerungsmittel sind z.B. Titandioxid, Bariumsulfat und/oder Eisenoxid besonders geeignet.

Die aus den erfindungsgemässen Pellets hergestellten Darreichungsformen, wie solche hierin genannt sind, z.B. die Tabletten oder Kapseln, enthalten den Wirkstoff oder die Wirkstoffe vorzugsweise in einer Menge von 25 bis 200 mg Wirkstoff pro Einheit der Darreichungsform, so beispielsweise in Mengen von 25 mg, 30 mg, 50 mg, 60 mg, 80 mg, 90 mg, 100 mg, 180 mg und 200 mg pro Einheit der Darreichungsform.

Die erfindungsgemässen Pellets stellt man beispielsweise her, indem man mindestens einen der genannten Wirkstoffe in mikronisierter Form mit den Zusatzstoffen mischt und in an sich bekannter Weise pelletiert und die erhaltenen Pellets gegebenenfalls mit einem Überzug, gegebenenfalls mit einem magensaftresistenten Lacküberzug, überzieht.

Die Herstellung von flüssigen Arzneiformen, wie Lösungen, Emulsionen sowie Suspensionen ist aus analogen Rezepturen bekannt. Als Lösungen werden hierin gemäss einschlägiger Literatur molekulardisperse Lösungen mit durchschnittlichen Teilchengrössen von weniger als 1nm (1 Nanometer) (Teile <1nm) bezeichnet. Kolloiddisperse Lösungen sind flüssige Systeme mit einer durchschnittlichen Teilchengrösse von 1 nm - 1 µm.

Zusatzstoffe für flüssige Darreichungsformen, wie Lösungen, sind an sich bekannt, wie z.B. Lösungsvermittler, wie Alkohole, vorzugsweise Ethanol; an sich bekannte Tenside; Viskositätserhöher, wie Cellulosen, Cellulosederivate, wie HPMC; Polyvinylverbinungen, wie Polyvinylalkohole, Polyvinylpyrrolidone, Polyvinylacetatphthalate; Polyethylenglykole, Polyethylenoxide; Polysaccharide, wie z.B. Polydextrose; Mucoadhäsivstoffe, wie Carrageenan und Chitosan; Silikate; Alginate, wie Alginsäure oder Natriumalginat; sowie Konservierungsmittel. Der Fachmann kann diese Stoffe ohne weiteres in den notwendigen Konzentrationen und Zusammensetzungen verwenden und optimieren. Sirupe enthalten oft bis zu 65 Gew.-% Zucker, z.B. Saccharose, sowie zusätzlich auch Geschmackskorrigentien.

Beispiele für bevorzugte Hilfsstoffe bzw. Zusatzstoffe für Emulsionen sind: Tenside, wie anionenaktive Tenside, Salz der Gallensäuren, vorzugsweise Natriumglycocholat, Arabisches Gummi; an sich bekannte kationenaktive und nichtionogene Tenside; höhere Fettalkohole, wie Cetylalkohol, Stearylalkohol; Partialfettsäureester mehrwertiger Alkohole, wie z.B. Ethylenmonostearat; Partialfettsäureester des Sorbitans (0.01-15%, bezogen auf das Gesamtgewicht der Formulierung), z.B. Sorbitanmonolaurat; Partialfettsäureester des Polyoxyethylensorbitans(0.1-15% bezogen auf Gesamtgewicht der Formulierung), z.B. Polyoxyethylensorbitanfettsäureester; Macrogolglycerinfettsäureester, wie Macrogolglycerollaurat; Fettsäureester des Polyoxyethylens (0.5-10%, bezogen auf das Gesamtgewicht der Formulierung); Fettalkoholether des Polyoxyethylens, z.B. Polyoxyethylenstearylether (0.5-25%, bezogen auf das Gesamtgewicht der Formulierung), Fettsäureester des Polyglycerols, z.B. Polyglycerololeat. Von den amphoteren Emulgatoren ist Lecithin bevorzugt. In Suspensionen sind Tenside als Dispergiermittel bevorzugt. Die verwendeten Konzentrationen sind dem Fachmann aus analogen Anwendungen bekannt.

### Beispiel 1 (Herstellung von mikronisiertem Indometacin und Acemetacin)

Grobkörniges Indometacin und Acemetacin wurden jeweils in pharmazeutischer Reinheit mit der Apparatur Chrispro® Jet-Mill MC 300KX-TD der Firma Micro-Macinazione SA nach dem folgendem Mahlprinzip mikronisiert: Das Mahlgut wird im Mahlbereich durch seitliche Düsen mit hoher Geschwindigkeit (Eingangspressdruck 6.0 bar; Arbeitspressdruck 4.0 bar, Düsenwinkel 32°5') aufeinander geschossen. Durch Zentrifugalwirkung wird das Mahlgut in Fein- und Grobgut getrennt. Das Feingut wird durch den zentralen Auslass ausgetragen, das Grobgut weiter solange zerkleinert, bis alles den geforderten Zerkleinerungsgrad erreicht hat. Es wurde ein Pulver mit einer Korngrössenverteilung gemäss Tabelle 1 erhalten:

**Tabelle 1**

| Kumulative Verteilung (%) | Korngrösse (pm) |
|---|---|
| 99 | 20.5 |
| 95 | 15.0 |
| 90 | 12.3 |
| 85 | 10.5 |
| 80 | 9.0 |
| 70 | 7.1 |
| 50 | 3.87 |
| 10 | 0.9 |

### Beispiel 2 (Herstellung einer Tablette)

Das in Beispiel 1 hergestellte mikronisierte Acemetacin bzw. Indometacin, die 4-fache Menge an modifizierter Lactose (sprühgetrocknet zur Direkttablettierung) und 0.3 Gew.-% Thaumatin (bezogen auf die Gesamtmasse) werden durch ein Sieb der Grösse 0.8 mm gesiebt und anschliessend gemischt. Nach Zugabe von 0.25 Gew.-% Magnesiumstearat wird erneut gemischt. Die resultierende Pulvermischung wird anschliessend auf einer Rundläuferpresse zu Tabletten verpresst.

### Beispiel 3 (Herstellung eines Granulats)

Das in Beispiel 1 hergestellte mikronisierte Acemetacin bzw. Indometacin wird mit 15% Zitronensäure und 74.6% Natriumsulfat (jeweils bezogen auf die Gesamtmasse des Granulates) gemischt. Der Farbstoff (0.075 Gew.-% bezogen auf das Gewicht der Gesamtmasse) wird in einer dreifachen Menge Wasser und Macrogol-Glycerolhydroxystearat (1.5 Gew.-% bezogen auf die Gesamtmasse) in einer 2,5-fachen Menge Ethanol gelöst. Mit der ethanolischen Lösung wird das oben genannte Pulvergemisch befeuchtet und anschliessend die wässrigen Farbstofflösung zugegeben. Bis zur gleichmässigen Befeuchtung wird noch genügend Ethanol/- Wasser-Gemisch 1:1 zugesetzt und anschliessend durch ein Sieb (2.0 mm) granuliert. Das Feuchtgranulat wird während ca. 2 Stunden bei 50°C bis zu einem Trocknungsverlust von weniger als 0.3 Gew.-% getrocknet und anschliessend durch ein Sieb der Maschengrösse 1.0 mm passiert.

### Beispiel 4 (Pulvermischung für Hartgelatinekapseln)

Das in Beispiel 1 hergestellte mikronisierte Acemetacin bzw. Indometacin werden mit Lactose (200 mesh, 17 Gew.-% bezogen auf die Gesamtmasse), sowie granulierter Lactose (35.7 Gew.-% bezogen auf die Gesamtmasse) gemischt. Nach Zugabe von 2 Gew.-% Talk und 2 Gew.-% Magnesiumstearat (bezogen auf die Gesamtmasse) und 0.35 Gew.-% Siliziumdioxid (bezogen auf die Gesamtmasse) wird die Pulvermischung in Hartgelatinekapseln abgefüllt.

### Beispiel 5 (Herstellung von Tabletten)

Polyvinyl-pyrrolidon (1 Gew.-% bezogen auf die Gesamtmasse) wird in der 70-fachen Menge Ethanol gelöst. Das in Beispiel 1 hergestellte mikronisierte Acemetacin bzw. Indometacin werden mit Lactose (46 Gew.-% bezogen auf die Gesamtmasse) und je 0.2 Gew.-% Neohesperidin und Thaumatin gemischt, mit der ethanolischen Polyvinylpyrrolidon-Lösung granuliert und anschliessend bei 40°C bis zu einem Restfeuchtgehalt von 5 Gew.-% getrocknet. Zu diesem Granulat werden 12.4 Gew.-% (bezogen auf die Gesamtmasse) Talk, 2.7 Gew.-% Weizenstärke und 1.5 Gew.-% Stearinsäure zugegeben und gemischt. Nach Zugabe von 1 Gew.-% Magnesiumstearat (bezogen auf die Gesamtmasse) und erneutem Mischen wird die Gesamtmasse auf einer Rundläuferpresse zu Tabletten verpresst.

### Beispiel 6 (Herstellung einer Brausetablette)

(a) Der Brausekörper: 32.5 Gew.-% Zitronensäure wasserfrei (Gew.-% jeweils bezogen auf die Gesamtmasse), 11.4 Gew.-% Natriumhydrogencarbonat, 19.8 Gew.-% Lactose-monohydrat, 6.5 Gew.-% Sorbitol und Neohesperidin DC (0.3 Gew.-%), 0.6 Gew.-% Aspartam und 0.12 Gew.-% Saccharin-Natrium , werden in den Granulierer geladen und intensiv gemischt. Gelborange (0.02 Gew.-%) werden in einer 3-fachen Menge Wasser von 50°C gelöst. Polyvinyl-pyrrolidon (0.4 Gew.-%) in einer ca. 5-fachen Menge Ethanol 96% gelöst. Mit dieser ethanolischen Lösung wird das oben genannte Pulvergemisch befeuchtet. Unmittelbar danach wir die Farblösung zugegeben. Bis zur gleichmässigen Befeuchtung wird noch Ethanol (96%ig) zugesetzt und anschliessend durch ein 2.0 mm Sieb granuliert. Das Feuchtgranulat wird bei 60°C bis zu einem Trocknungsverlust von 0.3 Gew.-% getrocknet und hierauf durch 1,25 mm Sieb passiert.
(b) Natriumhydrogencarbonat, siliconisiert: In einem Feuchtmischgerät wird Natriumhydrogencarbonat (1.3 Gew.-% bezogen auf die Gesamtmasse) vorgelegt. Unter ständigem Rühren wird dieser Trockensubstanz die entsprechende Menge (0.005% bezogen auf die Gesamtmasse) Silicon-Antischaumemulsion (33%-ig) langsam zugesetzt. Es wird etwa 30 Minuten nachgerührt und danach auf Horden bei 55°C getrocknet und durch ein 0.7 mm Sieb gesiebt.
(c) die Brausetablette: Der oben beschriebene Brausekörper [Absatz (a)], das oben beschriebene siliconisierte Natriumhydrogencarbonat [Absatz (b)], die in Beispiel 3 hergestellten Pellets (20.6 Gew.-%; jeweils bezogen auf die Gesamtmasse), sowie 1.8 Gew.-% Natriumhydrogencarbonat, 3.1 Gew.-% Polyethylenglycol 6000 und 1.5 Gew.-% Citronenaroma werden in einem geeigneten Freifallmischer während 15 Minuten gemischt. Die fertige Mischung wird bei einer relativen Feuchte von maximal 25% bei 20°C auf einer Rundläuferpresse zu Brausetabletten verpresst.

### Beispiel 7 (Herstellung eines Brausepulvers)

(a) Der Brausekörper: 32.5 Gew.-% Zitronensäure wasserfrei (Gew.-% jeweils bezogen auf die Gesamtmasse), 11.4 Gew.-% Natriumhydrogencarbonat, 19.5 Gew.-% Lactose-monohydrat, 6.5 Gew.-% Sorbitol und Neohesperidin DC (0.3 Gew.-%), Thaumatin (0.3 Gew.-%), 0.6 Gew.-% Aspartam und 0.12 Gew.-% Saccharin-Natrium , werden in den Granulierer geladen und intensiv gemischt. Gelborange (0.02 Gew.-%) werden in einer 3-fachen Menge Wasser von 50°C gelöst. Polyvinylpyrrolidon (0.4 Gew.-%) in einer ca. 5-fachen Menge Ethanol 96% gelöst. Mit dieser ethanolischen Lösung wird das oben genannte Pulvergemisch befeuchtet. Unmittelbar danach wir die Farblösung zugegeben. Bis zur gleichmässigen Befeuchtung wird noch Ethanol (96%ig) zugesetzt und anschliessend durch ein 2.0 mm Sieb granuliert. Das Feuchtgranulat wird bei 60°C bis zu einem Trocknungsverlust von 0.3 Gew.-% getrocknet und hierauf durch 1,25 mm Sieb passiert.
(b) Natriumhydrogencarbonat, siliconisiert: In einem Feuchtmischgerät wird Natriumhydrogencarbonat (1.3 Gew.-% bezogen auf die Gesamtmasse) vorgelegt. Unter ständigem Rühren wird dieser Trockensubstanz die entsprechende Menge (0.005% bezogen auf die Gesamtmasse) Silicon-Antischaumemulsion (33%-ig) langsam zugesetzt. Es wird etwa 30 Minuten nachgerührt und danach auf Horden bei 55°C getrocknet und durch ein 0.7 mm Sieb gesiebt.
(c) fertiges Brausepulver: Der oben beschriebene Brausekörper [Absatz (a)], das oben beschriebene siliconisierte Natriumhydrogencarbonat [Absatz (b)], die in Beispiel 3 hergestellten Pellets (20.6 Gew.-%; jeweils bezogen auf die Gesamtmasse), sowie 1.8 Gew.-% Natriumhydrogencarbonat, 3.1 Gew.-% Polyethylenglycol 6000 und 1.5 Gew.-% Citronenaroma werden in einem geeigneten Freifallmischer während 15 Minuten gemischt. Die fertige Mischung wird bei einer relativen Feuchte von maximal 25% bei 20°C als Brausepulver als Einzeldosis z.B. in ein Sachet verpackt.

### Beispiel 8 (Herstellung von Brausetabletten und Brausepulver)

In analoger Weise zu Beispiel 6 für die Herstellung einer Brausetablette und in analoger Weise zu Beispiel 7 für die Herstellung von Brausepulver können mit den in Tabelle 2 angegebenen Zusammensetzungen Brausetabletten und Brausepulver hergestellt werden, unter Verwendung von mikronisiertem Acemetacin. Die angegebene Menge an Acemetacin ist entsprechend gegen Indometacin austauschbar.

**Tabelle 2**

| | Formulierung 1 (mg) | Formulierung 2 (mg) |
|---|---|---|
| Acemetacin (Wirkstoff) | 60.00 | 60.00 |
| Hilfsstoffe: | | |
| Zitronensäure, wasserfrei | 1156.00 | 1156.00 |
| Natriumhydrogencarbonat | 470.34 | 470.34 |
| Natriumsulfat | 500.00 | 500.00 |
| alpha-Lactose x 1 Hz0 | 642.30 | 642.30 |
| Sorbitol | 212.00 | 212.00 |
| Gelborange 85 E 110 | 1.20 | 1.20 |
| Macrogol-Glycerolhydroxystearat | 10.00 | 10.00 |
| Polyvidon | 14.00 | 14.00 |
| Neohesperidin DC, E 959 | 10.00 | -.- |
| Thaumatin, E. 957 | -.- | 10.00 |
| Aspartam | 20.00 | 20.00 |
| Sacharin-Natrium | 4.00 | 4.00 |
| Zitronen-Aroma, Evogran, 301686, Symrise | 50.00 | 50.00 |
| Polyethylenglycol 6000 | 100.00 | 100.00 |
| Silicon Antischaumemulsion | 0.16 | 0.16 |

## Patentansprüche

1. Pharmazeutisch wirksame Zusammensetzung in einer pharmazeutischen peroralen Darreichungsform, welche mindestens einen der Wirkstoffe Indometacin oder Acemetacin sowie gegebenenfalls weitere Zusatzstoffe enthält, **dadurch gekennzeichnet,**
(i) **dass** diese Zusammensetzung den Wirkstoff, oder ein Gemisch dieser Wirkstoffe, in mikronisierter Form enthält, wobei diese mikronisierte Form durch Mikronisierung mit mechanischen Mitteln erhalten wurde;
(ii) **dass** mindestens 90 Vol.-% des mikronisierten Wirkstoffs eine mittlere Korngrössenverteilung von weniger als 25 µm (Mikron) aufweist und die untere Grenze der Korngrössenverteilung bei 0.1 µm (Mikron) liegt;
(iii)wobei dieser mikronisierte Wirkstoff in Form von Mikrokristallen vorliegt; und
(iv) diese Zusammensetzung den Wirkstoff oder die Wirkstoffe im Gemisch mit mindestens einem Flavonoid-Derivat enthält, welches ausgewählt ist aus der Gruppe enthaltend Chalconglycoside und Dihydrochalconglycoside.

2. Zusammensetzung nach Anspruch 1, **dadurch gekennzeichnet, dass** der mikronisierte Wirkstoff, oder das Gemisch der mikronisierten Wirkstoff durch Trockenmahlung, Luftstrahlmahlung (jet milling) oder Nassmahlung, vorzugsweise durch Luftstrahlmahlung erhalten wurde.

3. Zusammensetzung nach einem der Ansprüche 1 oder 2, **dadurch gekennzeichnet, dass** diese perorale Darreichungsform in Form eines losen Pulvers, als Peroraltabletten, Kautabletten, Oraltabletten, Lösungstabletten, Brausetabletten; gefüllt in Kapseln, vorzugsweise in Hartgelatinekapseln oder Weichgelatinekapseln; oder in einer flüssigen Arzneiform, vorliegt.

4. Zusammensetzung nach einem der Ansprüche 1-3, **dadurch gekennzeichnet, dass** mindestens 50 Vol.-% des Wirkstoffs eine mittlere Verteilung der Korngrösse von weniger als 10 µm (Mikron) aufweist und die untere Grenze der Korngrössenverteilung bei 0.1 µm (Mikron) liegt.

5. Zusammensetzung nach einem der Ansprüche 1-3, **dadurch gekennzeichnet, dass** mindestens 30 Vol.-% des Wirkstoffs eine mittlere Korngrössenverteilung von weniger als 5 µm (Mikron) aufweist und die untere Grenze der Korngrössenverteilung bei 1 µm (Mikron) liegt.

6. Zusammensetzung nach Anspruch 1, **dadurch gekennzeichnet, dass** die Flavonoid-Derivate ausgewählt sind aus der Gruppe enthaltend Naringinchalcon, Hesperitin-dihydrochalcon-glucosid, Neohesperidin-dihydrochalcon, und Neohesperidin-dihydrochalcon.

7. Zusammensetzung nach einem der Ansprüche 1 oder 2, **dadurch gekennzeichnet, dass** das Gewichtsverhältnis von Wirkstoff zur Flavonoidverbindung im Bereich von 10:1 bis 50:1, liegt.

8. Zusammensetzung nach einem der Ansprüche 1 oder 2, **dadurch gekennzeichnet, dass** das Gewichtsverhältnis von Wirkstoff zur Flavonoidverbindung im Bereich von 2:1 bis 50:1 liegt.

9. Zusammensetzung nach einem der Ansprüche 1 oder 2, **dadurch gekennzeichnet, dass** das Gewichtsverhältnis von Wirkstoff zu Neohesperidin-dihydrochalcon bei 6:1 liegt.

10. Zusammensetzung nach Anspruch 3, in Form einer Brausetablette, **dadurch gekennzeichnet, dass** diese aus (a) einem Wirkstoffgranulat enthaltend den mikronisierten Wirkstoff, welcher (b) im Gemisch mit mindestens einer FlavonoidVerbindung vorliegt, und (c) einen Brausekörper bestehend aus mindestens einer silikonisierten anorganischen Carbonatverbindung oder Hydrocarbonatverbindung, sowie gegebenenfalls (d) weiteren Zusatzstoffen, besteht.

11. Zusammensetzung nach Anspruch 10, **dadurch gekennzeichnet, dass** die gegebenenfalls enthaltenen Zusatzstoffe ausgewählt sind aus der Gruppe enthaltend Süssstoffe, synthetische Zuckerersatzstoffe sowie deren Salze, Polyole, natürliche und synthetisch hergestellte Aromastoffe, Beschwerungsmittel, Tenside, Farbstoffe, Füllmittel, und Bindemittel.

12. Zusammensetzung nach Anspruch 1, **dadurch gekennzeichnet, dass** diese als Pellets vorliegt und diese (a) mindestens einen der Wirkstoffe Indometacin oder Acemetacin oder ein Gemisch derselben in mikronisierter Form und zusätzlich ein Bindemittel sowie ein Beschwerungsmittel enthalten, und gegebenenfalls (b) diese Pellets mit einem magensaft-resistenten Lack überzogen und/oder in Gegenwart eines magensaftresistenten Lacks granuliert worden sind.

13. Zusammensetzung nach Anspruch 12, **dadurch gekennzeichnet, dass** die Pellets eine scheinbare Dichte von 1,4-2,4 g/cm³, vorzugsweise von 1,5-1,8 g/cm³, vorzugsweise 1,5-1,8 g/cm³, aufweisen und deren Durchmesser im Bereich von 0,2-1,8 mm, liegt.

14. Zusammensetzung nach Anspruch 13, **dadurch gekennzeichnet, dass** die Pellets 0,1-80 Gew.-% Wirkstoff, gegebenenfalls 20-95 Gew.-%, vorzugsweise etwa 40-80 Gew.-%, Beschwerungsmittel und im Weiteren Bindemittel, Farbstoff und Säuerungsmittel, ad 100 Gew.-% enthalten.

15. Die aus den Pellets gemäss einem der Ansprüche 12-14 hergestellten Darreichungsformen, welche den Wirkstoff oder die Wirkstoffe in einer Menge von 25 mg bis 200 mg Wirkstoff pro Einheit der Darreichungsform enthalten, vorzugsweise in Mengen von 25 mg, 30 mg, 50 mg, 60 mg, 80 mg, 90 mg, 100 mg, 180 mg und 200 mg pro Einheit der Darreichungsform.

16. Verwendung der Zusammensetzung nach Anspruch 1, zur Herstellung einer Darreichungsform für die medizinische Behandlung von chronischen und akuten Schmerzzuständen, Entzündungen und Fieber, insbesondere Chronische Polyarthritis, Degenerative Gelenkerkrankungen, insbesondere der großen Gelenke und der Wirbelsäule, Bechterew-Erkrankung, Gicht, Entzündliche Zustände der Gelenke, Muskeln und Sehnen, Sehnenscheidenentzündungen, Schleimbeutelentzündungen, Hexenschuss (Lumbago), Entzündungen oberflächlicher Venen (Thrombophlebitis).

## Claims

1. Pharmaceutically active composition in a pharmaceutical peroral dosage form, which contains at least one of the active substances indometacin or acemetacin and optionally further additives, **characterized in that**:
(i) this composition contains the active substance, or a mixture of these active substances, in micronized form, this micronized form being obtained through micronization by mechanical means;
(ii) at least 90 vol.% of the micronized active substance has an average particle size distribution of less than 25 µm (microns) and the lower limit of the particle size distribution is 0.1 µm (microns);
(iii) this micronized active substance is in the form of microcrystals; and
(iv) this composition contains the active substance or the active substances in a mixture with at least one flavonoid derivative selected from the group including chalcone glycosides and dihydrochalcone glycosides.

2. Composition according to claim 1, **characterized in that** the micronized active substance, or the mixture of the micronized active substances, was obtained by dry milling, jet milling or wet milling, preferably by jet milling.

3. Composition according to either claim 1 or claim 2, **characterized in that** this peroral dosage form is present in the form of a loose powder, peroral tablets, chewable tablets, oral tablets, soluble tablets or effervescent tablets, is filled into capsules, preferably hard gelatin capsules or soft gelatin capsules, or is present in a liquid dosage form.

4. Composition according to any of claims 1 to 3, **characterized in that** at least 50 vol.% of the active substance has an average particle size distribution of less than 10 µm (microns) and the lower limit of the particle size distribution is 0.1 µm (microns).

5. Composition according to any of claims 1 to 3, **characterized in that** at least 30 vol.% of the active substance has an average particle size distribution of less than 5 µm (microns) and the lower limit of the particle size distribution is 1 µm (micron).

6. Composition according to claim 1, **characterized in that** the flavonoid derivatives are selected from the group including naringin chalcone, hesperitin dihydrochalcone glucoside, neohesperidin dihydrochalcone, and neohesperidin dihydrochalcone.

7. Composition according to either claim 1 or claim 2, **characterized in that** the weight ratio of the active substance to the flavonoid compound is in the range of 10:1 to 50:1.

8. Composition according to either claim 1 or claim 2, **characterized in that** the weight ratio of the active substance to the flavonoid compound is in the range of 2:1 to 50:1.

9. Composition according to either claim 1 or claim 2, **characterized in that** the weight ratio of the active substance to the neohesperidin dihydrochalcone is 6:1.

10. Composition according to claim 3 in the form of an effervescent tablet, **characterized in that** it consists of (a) a granular active substance containing the micronized active substance, which (b) is in a mixture with at least one flavonoid compound, and (c) an effervescent body consisting of at least one siliconized inorganic carbonate compound or hydrocarbonate compound, and optionally (d) further additives.

11. Composition according to claim 10, **characterized in that** the optionally contained additives are selected from the group including sweeteners, synthetic sugar substitutes and the salts, polyols, natural and synthetically produced flavorings, weighting agents, surfactants, colorants, fillers and binders thereof.

12. Composition according to claim 1, **characterized in that** it is present as pellets and these contain (a) in micronized form at least one of the active substances indometacin or acemetacin or a mixture thereof and additionally a binder and a weighting agent, and optionally (b) these pellets have been coated with a gastric acid-resistant coating and/or granulated in the presence of a gastric acid-resistant coating.

13. Composition according to claim 12, **characterized in that** the pellets have an apparent density of 1.4 to 2.4 g/cm³, preferably 1.5 to 1.8 g/cm³, more preferably 1.5 to 1.8 g/cm³, and their diameter is in the range of 0.2 to 1.8 mm.

14. Composition according to claim 13, **characterized in that** the pellets contain 0.1 to 80 wt.% active substance, optionally 20 to 95 wt.%, preferably approximately 40 to 80 wt.%, weighting agent, and in addition binder, colorant and acidifier up to 100 wt.%.

15. Dosage forms prepared from the pellets according to any of claims 12 to 14, which contain the active substance or the active substances in an amount of 25 mg to 200 mg of active substance per unit of the dosage form, preferably in amounts of 25 mg, 30 mg, 50 mg, 60 mg, 80 mg, 90 mg, 100 mg, 180 mg and 200 mg per unit of the dosage form.

16. Use of the composition according to claim 1, for producing a dosage form for the medical treatment of chronic and acute pains, inflammations and fevers, in particular rheumatoid arthritis, degenerative arthritis, in particular in the large joints and the spine, ankylosing spondylitis, gout, inflammations of the joints, muscles and tendons, tenosynovitis, bursitis, lumbago, superficial thrombophlebitis.

## Revendications

1. Composition pharmaceutiquement active sous une forme posologique pharmaceutique à usage oral, laquelle contient au moins l'un des principes actifs que sont l'indométhacine ou l'acémétacine, et éventuellement d'autres additifs, **caractérisée**
(i) **en ce que** cette composition contient le principe actif ou un mélange de ces principes actifs sous forme micronisée, cette forme micronisée étant obtenue par micronisation par des moyens mécaniques ;
(ii) **en ce qu'**au moins 90 % en volume du principe actif micronisé présente une granulométrie moyenne inférieure à 25 µm (microns) et en ce que la limite inférieure de la granulométrie est de 0,1 µm (micron) ;
(iii) ledit principe actif micronisé se présentant sous forme de microcristaux ; et
(iv) ladite composition contenant le principe actif ou les principes actifs en mélange avec au moins un dérivé de flavonoïde, lequel est choisi dans le groupe contenant le glycoside de chalcone et le glycoside de dihydrochalcone.

2. Composition selon la revendication 1, **caractérisée en ce que** le principe actif micronisé ou le mélange des principes actifs micronisés a été obtenu par broyage à sec, broyage à jet d'air (broyage par jet) ou broyage par voie humide, de préférence par broyage à jet d'air.

3. Composition selon l'une des revendications 1 ou 2, **caractérisée en ce que** cette forme posologique à usage oral se présente sous forme d'une poudre de faible cohérence, comme comprimés à usage oral, comprimés à croquer, comprimés oraux, comprimés de solution, comprimés effervescents ; chargée dans des capsules, de préférence dans des capsules de gélatine dure ou dans des capsules de gélatine molle ; ou sous une forme pharmaceutique liquide.

4. Composition selon l'une des revendications 1 à 3, **caractérisée en ce qu'**au moins 50 % en volume du principe actif présente une granulométrie moyenne inférieure à 10 µm (microns) et **en ce que** la limite inférieure de la granulométrie est de 0,1 µm (micron).

5. Composition selon l'une des revendications 1 à 3, **caractérisée en ce qu'**au moins 30 % en volume du principe actif présente une granulométrie moyenne inférieure à 5 µm (microns) et **en ce que** la limite inférieure de la granulométrie est de 1 µm (micron).

6. Composition selon la revendication 1, **caractérisée en ce que** les dérivés de flavonoïdes sont choisis dans le groupe contenant la naringine chalcone, l'hespéritine dihydrochalcone glucoside et la néohespéridine dihydrochalcone.

7. Composition selon l'une des revendications 1 ou 2, **caractérisée en ce que** le rapport pondéral du principe actif au composé flavonoïde est compris entre 10:1 et 50:1.

8. Composition selon l'une des revendications 1 ou 2, **caractérisée en ce que** le rapport pondéral du principe actif au composé flavonoïde est compris entre 2:1 et 50:1.

9. Composition selon l'une des revendications 1 ou 2, **caractérisée en ce que** le rapport pondéral du principe actif à la néohespéridine dihydrochalcone est de 6:1.

10. Composition selon la revendication 3, sous forme d'un comprimé effervescent, **caractérisée en ce qu'**elle consiste en (a) un granulé de principe actif contenant le principe actif micronisé, lequel (b) est présent dans le mélange avec au moins un composé flavonoïde, et (c) un corps effervescent consistant en au moins un composé carbonate ou un composé hydrocarbonate inorganique siliconé, et éventuellement (d) d'autres additifs.

11. Composition selon la revendication 10, **caractérisée en ce que** les additifs éventuellement contenus sont choisis dans le groupe contenant les édulcorants, les faux sucres synthétiques ainsi que leurs sels, polyols, arômes naturels et produits synthétiquement, agents alourdissants, tensioactifs, colorants, charges et liants.

12. Composition selon la revendication 1, **caractérisée en ce qu'**elle se présente sous forme de pastilles et **en ce qu'**elle contient (a) au moins l'un des principes actifs que sont l'indométhacine ou l'acémétacine ou un mélange de ceux-ci sous forme micronisée, et en outre, un liant ainsi qu'un agent alourdissant, et éventuellement **en ce que** (b) ces pastilles ont été enrobées avec une laque résistant au suc gastrique et/ou granulées en présence d'une laque résistant au suc gastrique.

13. Composition selon la revendication 12, **caractérisée en ce que** les pastilles présentent une densité apparente de 1,4 à 2,4 g/cm³, de préférence de 1,5 à 1,8 g/cm³, préférablement de 1,5 à 1,8 g/cm³, et **en ce que** leur diamètre est compris entre 0,2 et 1,8 mm.

14. Composition selon la revendication 13, **caractérisée en ce que** les pastilles contiennent 0,1 à 80 % en poids du principe actif, éventuellement 20 à 95 % en poids, de préférence environ 40 à 80 % en poids d'agent alourdissant, et en outre, un liant, un colorant et un acidifiant, dans une proportion donnant 100 % en poids.

15. Formes posologiques préparées à partir des pastilles selon l'une des revendications 12 à 14, lesquelles contiennent le ou les principes actifs en une quantité de 25 à 200 mg du principe actif par unité de la forme posologique, de préférence en des quantités de 25 mg, 30 mg, 50 mg, 60 mg, 80 mg, 90 mg, 100 mg, 180 mg et 200 mg par unité de la forme posologique.

16. Utilisation de la composition selon la revendication 1, pour la préparation d'une forme posologique destinée au traitement médical de douleurs, d'inflammations et de la fièvre chroniques et aiguës, en particulier de la polyarthrite chronique, des maladies dégénératives des articulations, notamment des grandes articulations et de la colonne vertébrale, de la maladie de Bechterew, de la goutte, de maladies inflammatoires des articulations, des muscles et des tendons, des tendinites, des bursites, du lumbago, des inflammations des veines superficielles (thrombophlébite).
